# EUROPEAN PATENT SPECIFICATION

(11) **EP 2 566 547 B1**
(45) Date of publication and mention of the grant of the patent: **19.08.2015**
(21) Application number: 11720616.9
(22) Date of filing: 13.04.2011
(51) Int. Cl.: A61M 15/00

(54) **DRY POWDER INHALER MOUTHPIECE BUTTON**
TROCKENPULVERINHALATOR-MUNDSTÜCK
BOUTON D'EMBOUT BUCCAL POUR INHALATEUR À POUDRE SÈCHE

(30) Priority: 26.04.2010 TR 201003238; 20.04.2010 TR 201003091; 13.04.2010 TR 201002877
(43) Date of publication of application: 13.03.2013
(73) Proprietor: Sima Patent ve Lisanslama Hizmetleri Ltd.Sti., Esenler/Istanbul (TR)
(72) Inventor: Bilgic, Mahmut, 34173 Merter/Istanbul (TR)
(86) International application number: PCT/TR2011/000089
(87) International publication number: WO 2011/129789

(56) References cited:
- WO-A1-02/36189
- WO-A1-2009/003989
- GB-A- 2 447 560
- US-A1- 2009 078 252

## Description

### Field of the Invention

The present invention relates to an inhaler appropriate for delivering the medicament in dry powder form used in the treatment of respiratory diseases, particularly in asthma and chronic obstructive pulmonary disease (COPD). In addition, the present invention relates to a child resistant inhaler comprising a blister package appropriate for carrying the medicament in dry powder form.

### Description of the Prior Art

It is well known to use inhalers for delivering medicaments utilized in the treatment and prophylaxis of respiratory diseases by the inhalation route. Inhalation treatment is the most commonly preferred treatment method in these diseases as the inhalers provide ease of use; the medicaments have a more rapid onset of time resulting from local administration and they have fewer side effects. Inhalers have been designed in order to provide effective and sufficient delivery of the medicaments used in the treatment of respiratory diseases, particularly in asthma and chronic obstructive pulmonary disease. These inhalers vary according to their operating mechanisms and the physical form of the medicament to be delivered.

In the inhalers used to deliver medicaments in dry powder form, the medicament is carried in reservoirs, capsules or blisters packages. It is important to deliver each dose to the patient with exact accuracy and preciseness since the required medicament dose in the inhalation is very low.

The inhalers comprising blister packages are more advantageous than the other inhalers in terms of their preparing the accurately and precisely measured one dose of the dry powder medicament contained by each blister pocket of the blister package for inhalation in order to provide controlled dosing in response to each actuation of the device. Moreover, the inhalers comprising blister packages can be used repeatedly as it is not required to place a blister package carrying the dry powder medicament to be inhaled into the device before the inhalation.

Due to the fact that the medicaments in dry powder form which are utilized in inhalation treatment have strong effects even on very low amounts and have serious side effects, the accidental use of these medicaments by healthy people, particularly by children, might be dangerous. In addition, inadvertent actuation of the device out of inhalation time may cause the blisters to be vainly opened and the dry powder medicament in the blisters to spill into the device, which results in uncontrolled dose intake.

The inhalation device marketed under the trade mark Diskus® by GlaxoSmithKlein is one of the most well-known inhalers on the market. This device operates with a blister strip package in which the dry powder medicament is carried and a slide mechanism. According to this, the mouthpiece cover hiding the mouthpiece is slid and the mouthpiece and the slide are exposed before the device is used. In this device, the blister package is provided to be indexed by moving the sliding bar from one end to the other along the path it follows; the blister pocket is opened and the medicament in dry powder form becomes ready for inhalation. The necessity to slide the cover to move the slide can prevent inadvertent trigger of the device to an extent. However, it is required to produce more reliable devices to minimize or eliminate the errors and the problems which may result from the cover and the slide of said device to be easily moved by one hand.

Some of the devices present in the state of the art are summarized below;

WO 2009/003989 discloses an inhaler suitable for delivery of the medicament in dry powder form from a blister package composed of a plurality of blister pockets. Said device comprises a mouthpiece, a gear mechanism, a rotatable mouthpiece cover, a drive gear, a housing and a pawl to prevent movement of the mouthpiece cover.

US 2009/078252 discloses a fault sensing system for dry powder inhalers. The document mainly aims to detect breakage or some other sort of failure (such as stretching or delamination or tearing) of the blister strip to prevent the user from using the device although it no longer delivers the dry powder.

GB 2447560 discloses a dry powder inhaler wherein the device is actuated upon movement of the mouthpiece such that it is easy to use and the dose is not exposed to moisture and contamination from the air as te seal is broken only prior to use.

WO02/36189 discloses a medicament dispenser comprising; a body, a holder shaped to fit within said body and moveable relative to the body, a cassette containing medicament carrierwherein movement of the holder relative to the body results in movement of the cassette between a first position and a second position such that the cassette is reversibly removable from the holder when the cassette is in the second position.

When the state of the art is taken into account, it is seen that there is need for inhalers providing to realize safe inhalation which guarantees to eliminate the problems mentioned above. Therefore, said device relates to the safe inhaler which has been designed not to enable inadvertent and accidental actuation and provide to deliver the dry powder medicament to the patient with controlled dosing.

In another aspect, said invention has been designed as child resistant to prevent inadvertent use by children.

In another aspect, said invention relates to the inhaler appropriate for delivering dry powder medicament from blister package in order to provide efficient and sufficient delivery of medicament in dry powder form.

### Summary of the Invention

An inhaler suitable for delivery of the medicament in dry powder form according to the present invention comprising;
- a blister package composed of a plurality of blister pockets each of which comprises medicament in dry powder form and which are spaced at equal intervals;
- a mouthpiece enabling the patient to inhale the medicament in dry powder form from the opened blister;
- a gear mechanism enabling the blister package to index and the medicament in dry powder form to become ready for inhalation;
- a rotatable mouthpiece cover covering the mouthpiece and triggering the gear mechanism;
- a housing situated between the upper housing member and the lower housing member in which the blister package and the gear mechanism are enclosed
characterized in that said inhaler comprises stabilizing resilient covers that are connected with the upper and the lower housing members, and the movement of the mouthpiece cover is hindered by pawls under the stabilizing resilient covers on connection points that enable the mouthpiece cover to communicate with the gear mechanism interlock with the mouthpiece cover when the mouthpiece is completely covered by the mouthpiece cover.

The inhaler pertaining to the present invention is actuated by the movement of the mouthpiece cover. However, stabilizing resilient covers are utilized so as to prevent the actuation of the device when the mouthpiece cover is inadvertently rotated. When the mouthpiece is completely covered by the mouthpiece cover and the device is ready to be actuated, the pawls under the stabilizing resilient covers interlock with the mouthpiece cover in both sides of the device and prevents the cover to move unrestrainedly. To actuate the device, resilient parts of the stabilizing resilient covers should be manually pressed on in order to move the mouthpiece cover before inhalation. In other words, the device is actuated by rotating the mouthpiece cover while pressing on the resilient parts of the stabilizing resilient covers at the same time and one dose of the medicament in dry powder form is prepared for inhalation. Thus, inadvertent actuation is prevented as the actuation of the device requires skill and the coordination of the both hands.

According to the present invention, the inhaler is an easy-grip, manual device which is appropriate for delivering medicament in dry powder form.

The housing of the device of the present invention has been designed such that each component of the blister package and the gear mechanism which have a significant role in enabling the device to work properly is situated accurately and works harmoniously. To this end, the housing is divided into several compartments. The used portion and the unused portion of the blister package are accommodated in separated compartments in order to prevent the medicament in dry powder form remaining in the opened blister pocket spilling on the other components of the housing. Furthermore, the housing also comprise a beak which enables the blister package to be peeled and a manifold through which the dry powder medicament in the open blister passes before reaching the mouthpiece during inhalation. The housing can be in any appropriate shape while it is preferably elliptic or circular.

The upper and the lower housing members interlock with each other and enclose the housing in order to keep the housing and the gear mechanism fixed together. The mouthpiece cover hiding the mouthpiece is rotated by being slid on the upper and lower housing members. The grids on the surface of the lower and the upper housing members provide effective actuation by preventing the slipping of the finger while rotating the mouthpiece cover. The upper and the lower housing members can be in any appropriate shape which provides ease of use.

The mouthpiece cover hiding the mouthpiece of the device of the present invention has been designed such that it actuates the device. When the upper and the lower housing members of the device are joined together, engagement tabs on the inside surface of the lower housing member engage with engagement recesses on the inside surface of the upper housing member and the upper and lower housing members are fixed tightly. In addition, the protrusions on the upper and the lower housing members are joined end to end and form a restricted path where the mouthpiece cover rotates. Before each inhalation, both the mouthpiece is uncovered and one dose of the medicament in dry powder becomes ready for inhalation as one of the blister pockets is opened as a result of the mouthpiece cover's preferably being manually rotated along the path restricted by the joining of the protrusions on the upper and the lower housing members. The rotational path on which the cover moves is restricted on both ends by the protrusions of the upper and the lower housing members. The constant-distance path that the protrusions of the upper and the lower housing members define results in the mouthpiece cover's being rotated by a fixed angle in response to the each actuation of the device.

The mouthpiece cover of the device is joined with the gear mechanism by the drive gear via the connection points. One end of the drive gear passes through the center of the lower housing member and tightly joins with the mouthpiece cover in one connection point while the other end passes through the center of the upper housing member and tightly joins with the mouthpiece cover in the other connection point. For each end of the drive gear to make a fixed and tight connection with the connection points of the mouthpiece cover, one side cover is used for each end of the drive gear. The ends of the drive gear are carved for the ends of the side covers so the ends of the side covers can tightly engage with them from inside and hence the ends of the drive gear can tightly interlock with the ends of the side covers.. Inner faces of these carved parts in each end of the drive gear have a matching shape with the shapes of the ends of the side covers. These side covers that attach with the each end of the drive gear passes through the connection point and provide the mouthpiece cover to synchronize with the drive gear. Each connection point of the mouthpiece cover is a hole having a shape matching with the shape of the end of the side cover that passes through this connection point. Therefore, each of the side covers passes through one connection point of the mouthpiece cover as it engages with one end of the drive gear and this enables the mouthpiece cover to be connected to the drive gear by two ends of said drive gear in a tight and reliable way and to synchronize with the drive gear.

On each connection point of the mouthpiece cover, there is a stabilizing resilient cover. Extensions under the stabilizing resilient covers pass through holes on the upper or the lower housing member according to the position of the connection point and enable the stabilizing resilient covers that they connect with remain stable. The rotation of the mouthpiece cover is prevented from both sides as the pawl under each of the stabilizing resilient covers in both sides of the device interlocks with the mouthpiece cover. Before inhalation, the resilient parts of each stabilizing resilient cover which are compatible with the shape of the fingers are pressed to raise the pawls and releasing the mouthpiece cover in order to move the mouthpiece cover which actuates the device. Thus, the mouthpiece cover can easily be rotated when the resilient parts of each stabilizing resilient cover on both sides of the device which match with the shape of the fingers are pressed on. When the resilient parts of the stabilizing resilient covers are not pressed, the pawls under the stabilizing resilient covers do not allow the mouthpiece cover to move. In brief, the pawls under the stabilizing resilient covers prevent inadvertent actuation of the device.

In the device of the present invention, the possibility to encounter probable problems such as uncontrolled dosing resulting from inadvertent actuation of the device is eliminated as it requires skills and coordination of the both hands to press on the resilient parts of the stabilizing resilient covers while rotating the mouthpiece cover so as to actuate the device and it is not generally possible particularly for children under the age of 12 to present the skill and the capability to rotate the mouthpiece of said device.

The mouthpiece cover that actuates the device can be found in one of two positions. For the gear mechanism that indexes the blister package to be triggered, the mouthpiece cover should be moved from a first position to second position. When the mouthpiece cover is in the first position, it leans on the protruding part in one end of the rotational path. The mouthpiece is completely hidden when the mouthpiece cover is in the first position and the device is on standby mode. When the mouthpiece cover is in the second position, it leans on the protruding part on the other end of the rotational path and one dose of the medicament in dry powder form is prepared for inhalation upon the actuation of the device.

Each gear of the gear mechanism in the device according to the present invention directly or indirectly engages with each other. The drive gear, which is one of the components of the gear mechanism, provides the mouthpiece cover to trigger the gear mechanism. In each actuation of the device, the constant-angle rotational movement of the cover is transmitted by to gear of the indexing ratchet wheel which interlocks with the indexing gear via the drive gear. The indexing wheel synchronizes with the indexing ratchet wheel when the mouthpiece cover is switched from the first position to the second position. The gear of the indexing wheel is engaged with the winding wheel gear and the pinion gear and causes them to move as well. The mechanism wheel engages with the inside of the winding wheel gear via the arms of the mechanism wheel. The counter gear that engages with the small gear under the base gear rotates by means of the base gear that engages with the pinion gear and the counter gear with the movement of the mouthpiece cover.Therefore, with the rotation of the indexing wheel gear, both the lid sheet of the blister package is provided to be coiled on the wings of the winding wheel as the winding wheel is rotated by the mechanism wheel that engages with the winding wheel gear, and the counter gear that engages with the small gear under the base gear is provided to be rotated by means of the base gear that engages with the pinion gear. After the counter gear has been rotated, the new number of unused blisters can be seen through the display aperture.

According to the present invention, the indexing wheel may be another component of the gear mechanism and it provides the blister package to be indexed properly and the opened blisters to be positioned accurately. The recesses of the indexing wheel match with the shape of the blister package and the blister pockets of the blister package are received in these recesses in sequence while the indexing wheel rotates. The rotation angle of the indexing wheel depends on the number of the recesses of the indexing wheel. In each actuation of the inhalation device, the indexing wheel can be rotated by 15° to 120°. According to the invention, there are preferably 8 recesses on the indexing wheel. Therefore, the indexing wheel is supposed to rotate by 45°in response to each actuation of the device for the opened blister pocket to be positioned accurately. As the indexing wheel rotates by the same angle, the blister package is indexed to the same extent and the opened blister pocket is provided to be accurately positioned.

At least one stopper component is arranged in any suitable part of the device in order to provide the blister package to be precisely positioned. The stopper can be in any suitable shape to execute said task

The counter gear in the device pertaining to the present invention displays the number of the unused blister pockets remaining in the device. In response to the actuation of the device by the mouthpiece cover, the mouthpiece is uncovered, the blister package is indexed and one dose of the dry powder medicament is prepared for inhalation while the counter gear rotates as well. Thus, the movement of the mouthpiece cover leads to the mouthpiece cover to be uncovered; one dose of the dry powder medicament to be ready for inhalation after the blister pocket is opened as well as providing the counter gear to rotate and display the new value of the unused blister pockets remained.

On the counter gear, there exist numerals equal to the number of the blister pockets present in the device and they are spaced at equal angles. In a device comprising 60 doses, the angle between the numerals is approximately 5 degrees. The counter gear rotates as a result of the reflection of the rotation of the indexing wheel gear via the pinion gear and the base gear. In response to the each actuation of the device, rotation of the indexing wheel by the same angle each time due to the accurate transmission of the movement of the mouthpiece cover to the gear mechanism via the drive gear results in the rotation of the counter wheel approximately by the same angle as well and each numeral on the counter wheel is clearly seen through the display aperture on the upper housing member. Therefore, the patient is sure about the number of the unused blister pockets remaining in the device.

Before inhalation, the device is actuated by pressing on the resilient parts of the stabilizing resilient covers on both faces of the device and rotating the mouthpiece cover along the rotational path, and the drive gear which is joined with the both connection points of the mouthpiece cover via the side covers precisely transmits the movement of the mouthpiece cover to the indexing ratchet wheel with the help of the side covers upon the actuation of the device. The indexing ratchet wheel interlocks with the indexing wheel from inside via its arms and enables the indexing wheel to rotate 45° in each actuation of the device. Upon the indexing wheel's movement, the blister package is indexed and it is peeled by means of the beak present in the housing. As the indexing wheel gear engages with the winding wheel gear and the pinion gear, these gears move upon the indexing wheel's move, too. Since the mechanism wheel engages both with the winding wheel gear and the winding wheel, the rotation of the winding wheel gear causes the winding wheel to move and lid sheet of the blister package coils tightly on the winding wheel. The pinion gear engages with the base gear while the small gear under the base gear engages with the counter gear. In brief, the movement of the mouthpiece cover along the rotational path causes the blister package to be accurately positioned as a result of the rotation of the indexing wheel by 45 degrees and the number of the unused blisters remaining in the device is clearly seen through the display aperture as the counter gear rotates by 5 degrees.

The inhaler according to the present invention comprises a blister package composed of a plurality of blister pockets each of which comprises medicament in dry powder form and which are spaced at equal intervals. The blister package carries the medicament in dry powder form in one-dose portions and it is preferably a blister strip and it is preferably peelable. The blister pockets comprised in the blister package are spaced in equal intervals and each of them carries one dose of the medicament in dry powder form.

While the blister package is indexed on the indexing wheel, the beak on the housing peels the blister. Therefore, one dose dry powder medicament becomes ready for inhalation after the blister package is peeled open in each actuation of the device.

The base sheet of the blister package on which the blister cavities are spaced is accumulated in the separated compartment of the housing. The lid sheet that provides impermeability of the blister package, on the other hand, is coiled on the winding wheel which is one of the components of the gear mechanism positioned in the other side of the housing.

The blister opened by the beak is situated immediately under a manifold. Upon the inhalation by the patient, the airflow that enters the device through at least one air inlet on the upper housing member entrains the dry powder medicament in the opened blister pocket via the manifold to the mouthpiece and enables the delivery of said medicament to the patient. The air inlet on the upper housing member that allows the entry of air can be in any suitable shape and size that enables external air to enter the device easily and at convenient speed.

The air inlet that the external air flow passes through is preferably designed not to be close where the patient holds the device in order not to prevent the air flow. Furthermore, in order to deliver the required amount of the dry powder medicament in the opened blister to the patient, the air inlet is designed such that it allows the entry of the airflow through the air inlet at a convenient angle.

One end of the manifold communicates with the opened blister while the other end communicates with the mouthpiece. On the end of the manifold that communicates with the blister, at least two apertures with four sub-apertures are situated. Upon the inhalation of the patient, some of the airflow which enters through the air inlet passes through one of these apertures and entrains the medicament in dry powder form through the other aperture to the manifold. There is preferably a tapered channel between the manifold and the mouthpiece to connect these components to each other. The medicament in dry powder form that is entrained to the manifold with the airflow is delivered to the patient via the mouthpiece. The manifold can be in any appropriate shape though it is preferably at least 1mm.

The mouthpiece is designed to fit the mouth for the patient to comfortably inhale the medicament in dry powder form. According to the shape of the device, the mouthpiece can be in any suitable shape or size as well as being fixed or movable. Furthermore, it could be attached or unattached to the upper and/or the lower cover.

Each component of the device pertaining to the present invention can be made of any suitable material though they are preferably be made of plastic. These plastics can preferably be chosen from a group comprising the types of styrene acrylonitrile, polyoxymethylene acetale, acrylic-polymethylmetacrylate, cellulose acetate, polyetheretherketone, polyvinyl choloride, polyethylene, polypropylene, acrylonitrile butadiene styrene, polycarbonate, polyamide, polystyrene, polyurathane or fluoropolymer while it is preferably polyoxymethylene acetale. The plastic components can be manufactured by methods like injection molding. Moreover, each component of the device can be in any suitable color.

According to the present invention, the lid and the base sheets constituting the blister package preferably consist of a plurality of layers. Polymeric layers, aluminum foil and preferably fluoropoylmer film are among the layers that form the lid and the base sheet.

According to the present invention, the lid and base sheets composing the blister package are sealed very tightly by at least one of the methods comprising cold formed bonding, hot metal bonding, hot metal welding, radio frequency welding, laser welding or ultrasonic welding in order to provide impermeability, more preferably by cold formed bonding method. Since these cold formed bonding methods can be carried out at lower temperatures than hot sealing methods, they are the most appropriate methods to use in the case that the medicament carried in the blister is heat sensitive.

Fluoropolymer film is a polymeric film which is used in blister packs and provides excellent moisture barrier. This chemically inert polymeric film does not cause any change in the taste of the formulation when it is in contact with the dry powder formulation. In addition, it easily constitutes a layered structure with the other polymeric layers which are composed of various polymers. It is appropriate to be transacted with heat.

For preserving the stability of the dry powder formulation stored in the blister package, preferably at least one of the polymeric layers comprises at least one desiccant agent including silica gel, zeolite, alumina, bauxite, anhydrous calcium sulfate, activated carbon and clay which has the property of water absorption in order to decrease gas and moisture permeability of the layer.

According to the invention, the thickness of the aluminum foil in the lid and the base sheets of the blister package are preferably chosen to be in the range of 5 to 80 µm, more preferably in the range of 15 to 65 µm.

According to the invention, the polymeric layers in the lid and the base sheets of the blister pack are made of the same or different polymers. The thickness of these polymeric layers varies according to the type of the polymeric substance used and its properties while they are preferably in the range of 5-100 µm, more preferably in the range of 15-60 µm.

The polymers composing the polymeric layer are preferably selected from thermoplastics such as polyethylene, polypropylene, polystyrene, polyolefin, polyamide, polyvinyl chloride, polyurethane or synthetic polymers.

The blister pockets in the blister package can be in any appropriate shape. The plurality of blister pockets spaced at equal intervals on the base sheet of the blister package can be the same or different shape, structure or volume.

The reference numbers of the drawings added to exemplify the present invention and the detailed description of the invention according to these drawings are given below but the scope of the invention should not be limited to these drawings.

### Brief Description of the Drawings

Figure 1 is a perspective view of an inhaler according to the present invention;
Figure 2 is an exploded view of the inhaler of the invention;
Figure 3 is a perspective view of the blister pack for use with the inhaler pertaining to the invention;
Figures 4a and 4b are perspective views of the housing of the inhaler according to the invention;
Figures 5a and 5b are perspective views of upper and lower housing members of the inhaler according to the invention;
Figure 6a is a perspective view of the mouthpiece cover of the inhaler of the invention;
Figure 6b is an exploded view of the communication between the mouthpiece cover, the drive gear and the stabilizing resilient covers in the inhaler of the invention;
Figure 6c is a cross-sectional view of the communication between the mouthpiece cover, the drive gear and the stabilizing resilient covers in the inhaler of the invention;
Figure 6d is a cross-sectional view of the communication between the mouthpiece cover, the drive gear and the stabilizing resilient covers in the inhaler of the invention;
Figure 6e is an exploded view of the communication between the drive gear and the side covers in the inhaler of the invention;
Figure 6f is a cross-sectional view of the connection of the stabilizing resilient cover with the lower housing member in the inhaler of the invention;
Figures 7a-7c are cross-sectional views of the engagement of the gears composing the gear mechanism with each other in the inhaler of the present invention;
Figure 8 is a cross-sectional view of the blister package delaminating in course of operation of the inhaler of the present invention;
Figure 9 is a perspective view of the counter gear used in the inhaler of the present invention.

### Detailed Description of the Drawings

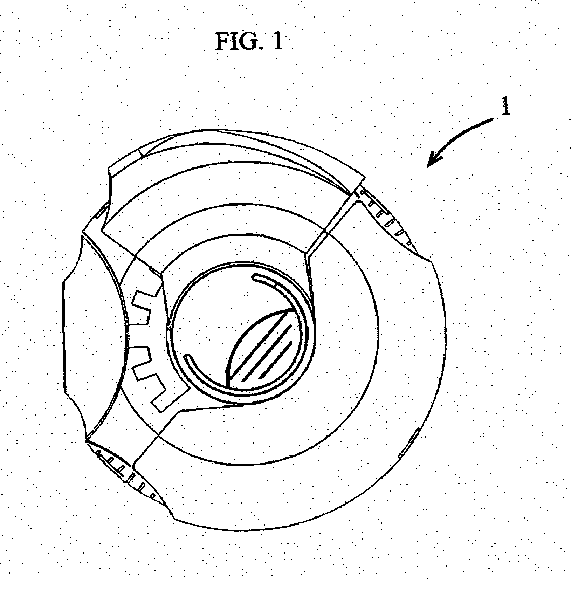

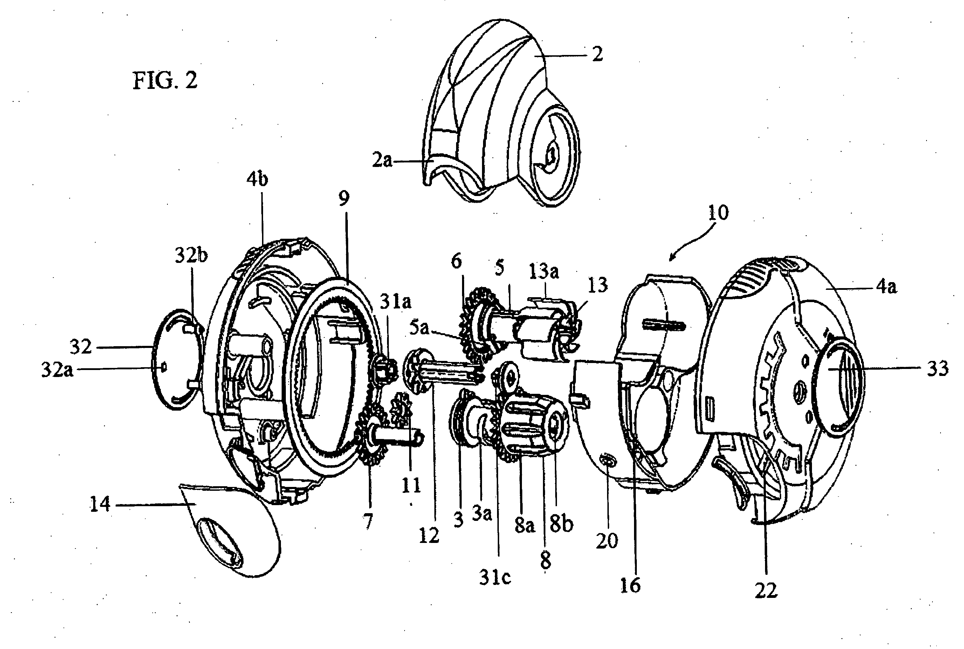

The inhaler (1) of the present invention comprises a gear mechanism situated in the housing (10) between the upper housing member (4a) and the lower housing member (4b) in order to enable the inhalation of the dry powder medicament carried in a blister package (15) as displayed in figures 1 and 2. Each component of the inhaler (1) is positioned at particular spots on the housing (10) to guarantee proper and accurate working.

The inhaler (1) of the present invention shown in Figure 1 is ready for inhalation. In this case, the mouthpiece cover (2) is in the first position and it completely covers the mouthpiece (14). The mouthpiece cover (2) has to be rotated by holding the carved part (2a) on one end of the mouthpiece cover (2) in order to switch to the second position from the first position wherein the mouthpiece is completely covered. In this way, the mouthpiece (14) is completely exposed when the mouthpiece cover (2) is switched from the first position to the second position and the gear mechanism is triggered by a drive gear (12). The drive gear (12) precisely transmits the movement of the mouthpiece cover (2) to an indexing ratchet wheel (3).

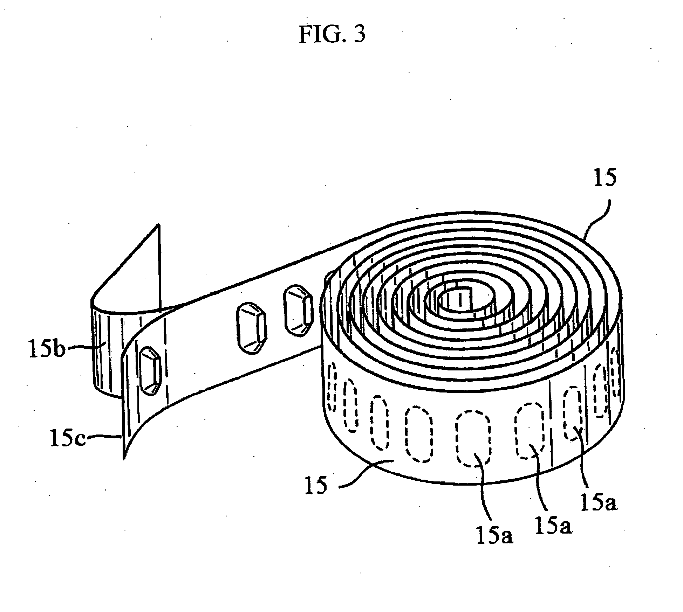

The indexing wheel (8) which engages with the indexing ratchet wheel (3) and enables the blister package (15) shown in figure 3 to be indexed. The blister pockets (15a) composing the blister package are received in the recesses (8a) on the indexing wheel and the blister package (15) is indexed when the indexing wheel (8) rotates. According to this, shapes of the recesses (8a) on the indexing wheel (8) have been designed to match the shapes of the blister pockets (15) composing the blister package (15) for the blister package to be indexed properly.

The blister package (15) shown in figure 3 is composed of the lid sheet (15b) which provides impermeability and the base sheet (15c) on which the blister pockets (15a) are spaced at equal intervals. Each blister pocket contains medicament in dry powder form comprising one or more active agents.

The rotational movement that the mouthpiece cover (2) of the device for actuation of the inhaler executes while switching from the first position to the second is transmitted to the indexing ratchet wheel (3) via the drive gear (12) that the mouthpiece cover (2) engages with. As displayed in figure 2, arms (3a) of the indexing ratchet wheel interlocks with protrusions (8b) inside the indexing wheel (8) and rotates the indexing wheel (8) unidirectionally. Therefore, the blister package (15) is indexed forward while the indexing wheel (8a) rotates as the blister pockets (15a) composing the blister package (15) are received in the recesses (8a) of the indexing wheel. The beak (16) in the housing (10) peel the blister package (15) while the blister package (15) is indexed and provides one blister pocket (15a) to be opened in response to each actuation of the device (1).

The winding wheel gear (6), which is another component of the gear mechanism, engages with the gear of the indexing wheel (8) as displayed in figure 2. The mechanism wheel (5) that interlocks the winding wheel (13) from inside has arms (5a) to interlock with the interior teeth of the winding wheel gear (6). Therefore, the mechanism wheel (5) engages both with the winding wheel (13) and the winding wheel gear (6) and moves with these component in actuation of the inhaler (1). When the indexing wheel (8) rotates the winding wheel gear (6), the winding wheel (13) rotates unidirectionally owing to the arms of the mechanism wheel (5a) interlocking with the interior teeth of the winding wheel gear (6) and the lid sheet (15b) which is peeled away by the beak (16) while the blister package is indexed is tightly coiled on the wings (13a) of the winding wheel. The base sheet (15c) of the blister package (15) where the blister pockets are spaced is accumulated in a separate part (18a) of the device.

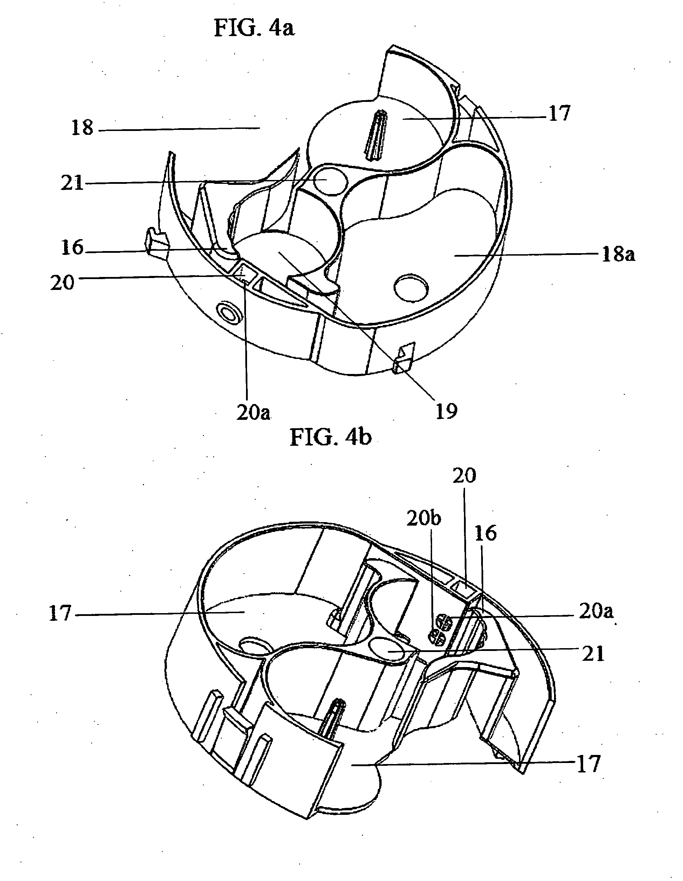

Different perspective views of the housing (10) wherein the gear mechanism and the other components of the inhaler (1) of the present invention are arranged are displayed in figures 4a and 4b. Furthermore, as can be seen in figures 4a and 4b, the housing (10) also comprises the components having significant roles in the actuation of the device such as the beak (16), the manifold (20), the apertures with four sub-apertures (20a, 20b). Each component comprised in the housing is situated in an appropriate part of the housing (10) in order to enable the inhaler (1) to work properly. The drive gear (12) passes through the center (21) of the housing and joins the mouthpiece cover (2) at two points. The blister package (15) is in the lower part (17) of the housing and coiled up. In response to each actuation of the device (1), the blister package (15) is peeled by the beak (16) in the housing while being indexed by the indexing wheel (8) situated in the upper part (19) of the housing. The lid sheet (15b) of the blister package (15) which provides impermeability is indexed over the beak (16) and coiled on the wings of the winding wheel (13a) which is situated in the side part (18) of the housing. The base sheet (15c) of the blister package (15) on which the blister pockets (15a) are spaced, on the other hand, is accumulated in the separated compartment (18a) of the housing (10). Upon the inhalation by the patient, air passes through the aperture with four sub-apertures (20a) under the manifold (20) and into the opened blister pocket; entrains the dry powder medicament contained in the opened blister pocket (15a) in response to each actuation of the device; and passes through the other aperture with four-sub-apertures (20b) and reach the mouthpiece via the manifold (20).

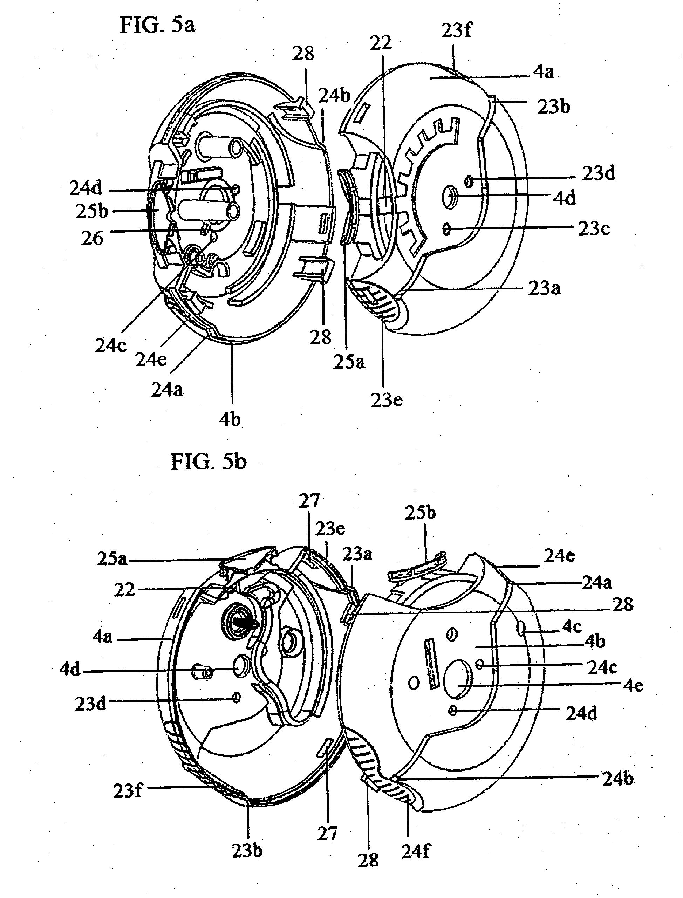

The housing (10) and the other components of the inhaler (1) of the present invention are kept together as upper housing member (4a) and lower housing member (4b) displayed in figures 5a and 5b are joined together. Engagement tabs (28) on the inside surface of the lower housing member (4b) engage with engagement recesses (27) on the inside surface of the upper housing member (4a) and the upper and lower housing members are fixed tightly. Therefore, the protrusions (23a, 23b) on the upper housing member (4a) and the protrusions (24a, 24b) on the lower housing member (4b) are joined end to end and define a restricted path for the rotational movement of the mouthpiece cover (2). In each actuation of the device, the constant-distance path that the protrusions (23a, 23b, 24a, 24b) of the upper and the lower housing members (4a, 4b) define allows said mouthpiece cover (2) to be rotated by a fixed angle. The mouthpiece cover (2) can be moved from the first position to the second position along this restricted path. When the mouthpiece cover (2) is in the first position, the mouthpiece (14) is completely covered, the device is in standby mode and the mouthpiece cover (2) leans on the first protrusion (23a) on the upper housing member and the first protrusion (24a) on the lower housing member. The mouthpiece (2) is manually slid along the rotational path with the help of the carved part (2a) to switch to the second position. The mouthpiece is completely exposed when the cover is in this position, one dose of the dry powder medicament is ready for inhalation and the mouthpiece cover (2) leans on the second protrusion (23b) on the upper housing member and the second protrusion (24b) on the lower housing member.

As displayed in figures 5a and 5b, one half (25a) of the tapered channel that interconnects the manifold (20) with the mouthpiece (14) is comprised in the upper housing member (4a) while the other half of it (25b) is comprised in the lower housing member (4b). The channel is constituted as a whole when the upper (4a) and the lower (4b) housing members are joined together. Upon the inhalation by the patient, the air that enters the device through at least one air inlet (22) arranged in the upper housing member (4a) passes through the aperture with four sub-apertures (20a), reaches the opened blister (15a) and entrains the dry powder medicament there to the manifold (20) by passing it through the other aperture with four sub-apertures (20b). Grids on the upper housing member (23e, 23f) and grids on the lower housing member (24e, 24f) prevent a slip of fingers when rotating the mouthpiece cover.

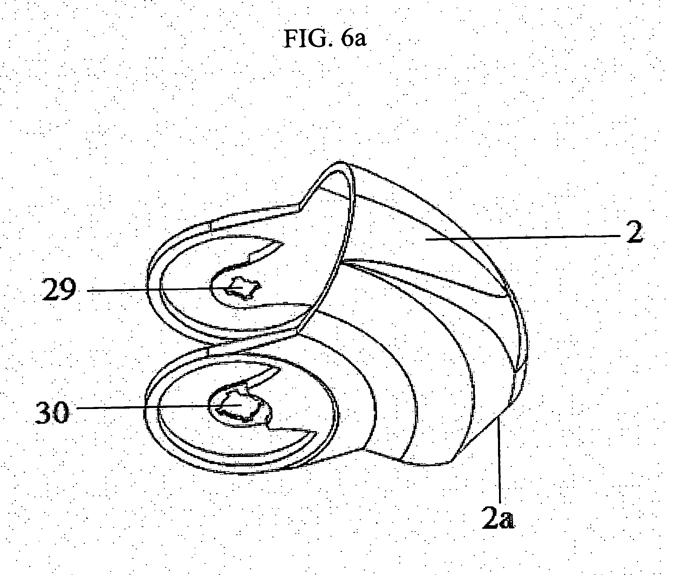

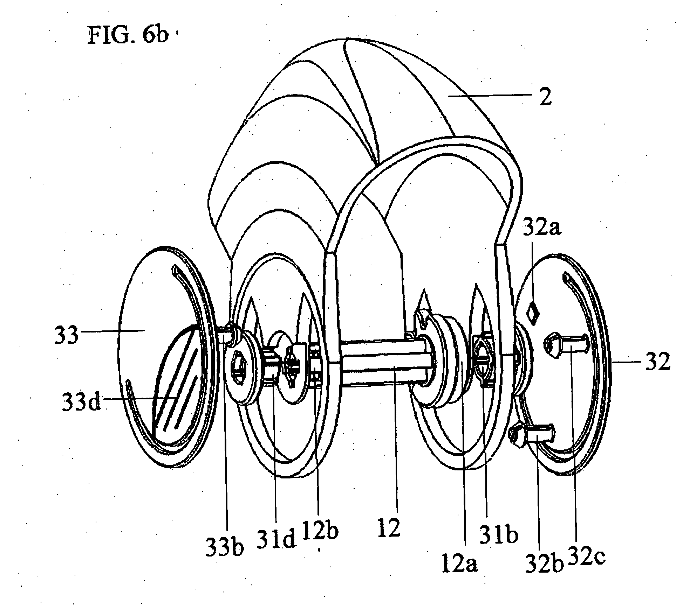

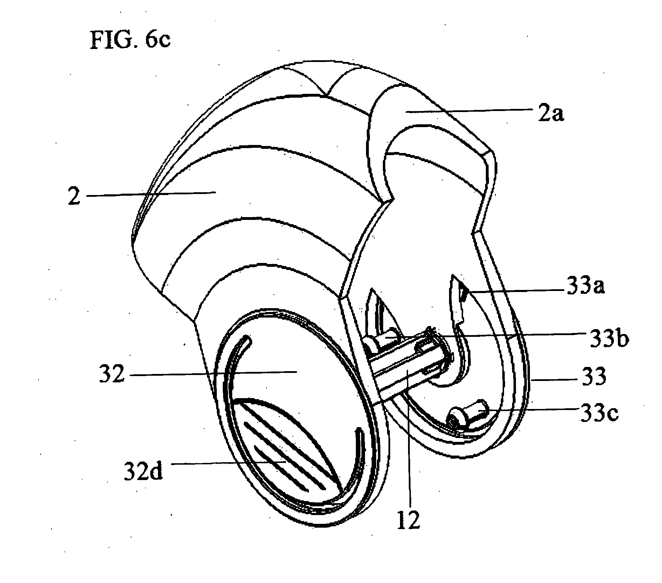

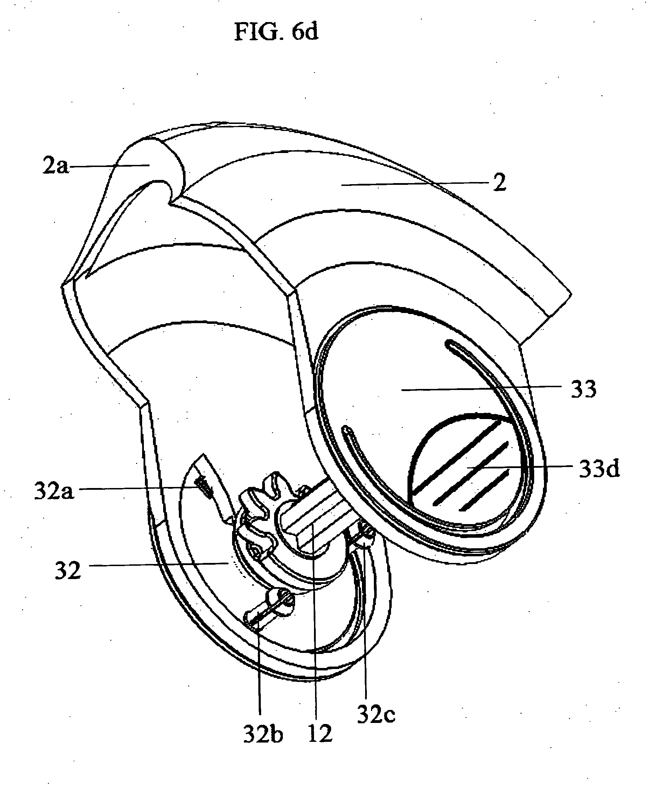

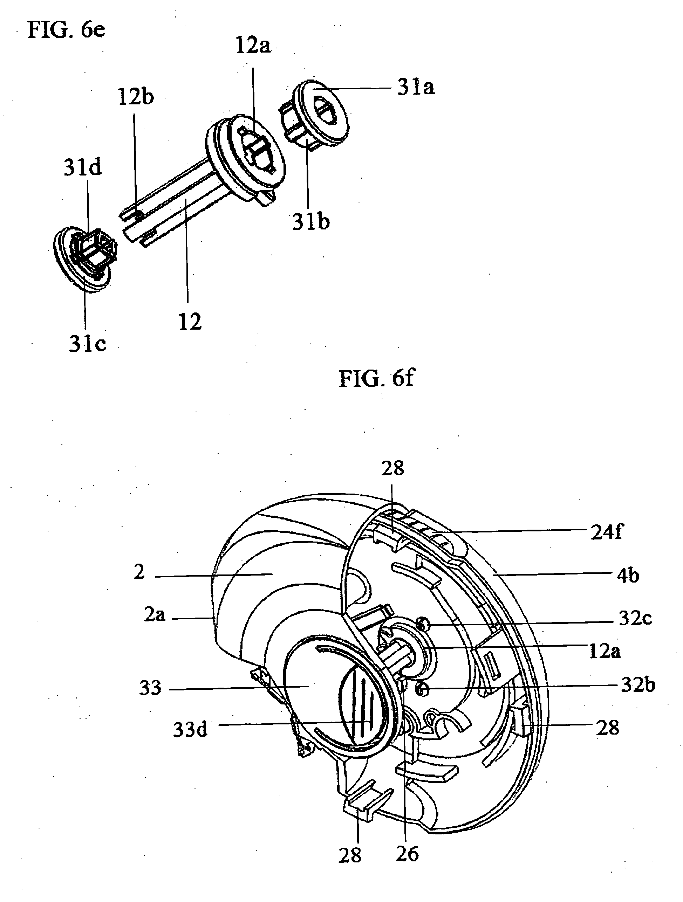

The mouthpiece cover (2) of the inhaler pertaining to the present invention is displayed in figure 6a. The carved part (2a) in one end of the device enables easily manuel movement of the mouthpiece cover (2). The mouthpiece cover (2) is joined with the gear mechanism via the connection points. The drive gear (12) is joined with the connection points (29, 30) of the mouthpiece cover via the side covers (31a, 31c) as can be seen in figures 6b, 6c and 6d illustrating the communication between the mouthpiece cover (2), the drive gear (12), side covers (31a, 31c) and stabilizing resilient covers (32,33). Each of these side covers (31a; 31c) passes through the center (4d) of the upper housing member or the center (4e) of the lower housing member and joins with the end (12a; 12b) of the drive gear. It can be seen in figure 6d that the both ends (12a; 12b) of the drive gear are carved such that the end of the side cover (31b; 31d) can pass through. Each end of the side covers (31d; 31b) passes through one of the connection points (29; 30) of the mouthpiece cover and it is received in the recess in one end (12b; 12a) of the drive gear, thus it tightly and stably interconnects the mouthpiece cover (2) with the drive gear (12). The mouthpiece cover (2) synchronizes with the drive gear (12) as the connection point (29; 30) of the mouthpiece cover which has a matching shape with the ends (31d; 31b) of the side covers that passes through it on both sides of the device and the end (12b; 12d) of the drive gear that it communicates with are on the same component.

As is seen from figures 6a-6e, the shapes of the ends (31b; 31d) of the side covers that are received in the carved parts on the ends of the drive gear and the shapes of the connection points (29, 30) of the mouthpiece cover are not identical since the two ends (12a, 12b) of the drive gear are not identical.

There is one stabilizing resilient cover (33; 32) on each connection point (29; 30) of the mouthpiece and on each side cover, as displayed in figures 2, 6a-6d and 6f. When the mouthpiece cover (2) is in the first position, the pawls (32a, 33a) under the stabilizing resilient covers, which are on the connection points (29, 30) of the mouthpiece, interlock with the mouthpiece cover (2) on both sides as seen in figures 6c and 6d. The pawl (33a) under the stabilizing resilient cover that is on the first connection point (29) interlocks with the mouthpiece cover (2) on one side (figure 6c). The pawl (32a) under the stabilizing resilient cover that is on the second connection point (30) of the mouthpiece cover (2) interlocks with the mouthpiece cover (2) on the other side (figure 6d). Thus, these pawls (32a, 33a) under the stabilizing resilient covers prevent the movement of the mouthpiece cover (2) and therefore the inadvertent actuation of the drive mechanism by interlocking with the mouthpiece on both sides.

Extensions (32b, 32c; 33b, 33c) under the stabilizing resilient covers pass through apertures (23c, 23d; 24c, 24d) on the upper and the lower housing members illustrated in figures 5a and 5b and keep the stabilizing resilient covers stable. Namely, the extensions (33b; 33c) under the stabilizing resilient cover that is on the first connection point (29) of the mouthpiece cover pass through the apertures (23c; 23d) on the upper housing member and provide the stabilizing resilient cover (33) to be stably joined with the device. The extensions (32b, 32c) under the stabilizing resilient cover on the second connection point (30) of the mouthpiece cover pass through the apertures (24c, 24d) on the lower housing member and provide the stabilizing resilient cover (32) to be stably joined with the device as illustrated in figure 6f.

Before inhalation, the resilient parts (32d, 33d) of each stabilizing resilient cover illustrated in figures 6c and 6d are pressed on to raise the pawls (32a, 33a) and release the mouthpiece cover (2) in order to actuate the gear mechanism of the device to prepare one dose of dry powder medicament before inhalation. The gear mechanism of the device is actuated and one blister pocket (15a) is opened for one dose of the dry powder medicament to be ready for inhalation when the resilient parts (32d, 33d) of the stabilizing resilient covers are pressed on and the mouthpiece cover (2) is switched from the first position to the second position simultaneously. The necessity to press on the resilient parts (32d, 33d) of the stabilizing resilient covers so as to actuate the gear mechanism preclude the consequences which may result from accidental and inadvertent actuations of the gear mechanism.

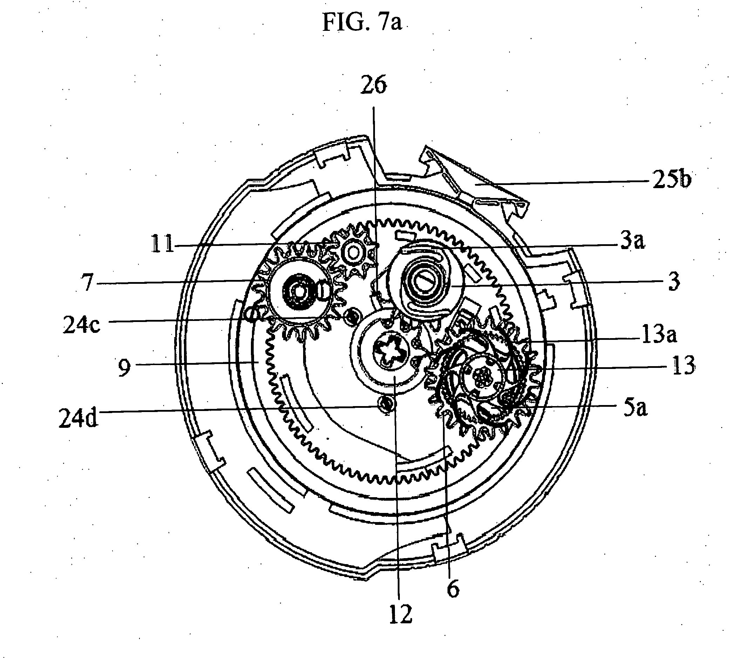

In figure 7a, it is displayed that a stopper (26) interlocks with the tooth of the indexing ratchet wheel (3) and hinders its rotation. The rotational movement of the mouthpiece cover (2) by the same angle in response to each actuation of the device (1) is accurately transmitted to the indexing ratchet wheel (3) by the drive gear (12) which is joined with the mouthpiece cover (2) on its both ends and the drive gear (12) is enabled to rotate by the same angle in each actuation of the device (1). The stopper component (26) in the lower housing member (4b) prevents backward movement of the blister package (15) indexed by the indexing wheel (8) which synchorizes with the indexing ratchet wheel by keeping the position of the indexing ratchet wheel (3) fixed and provides the blister package (15) to be precisely positioned.

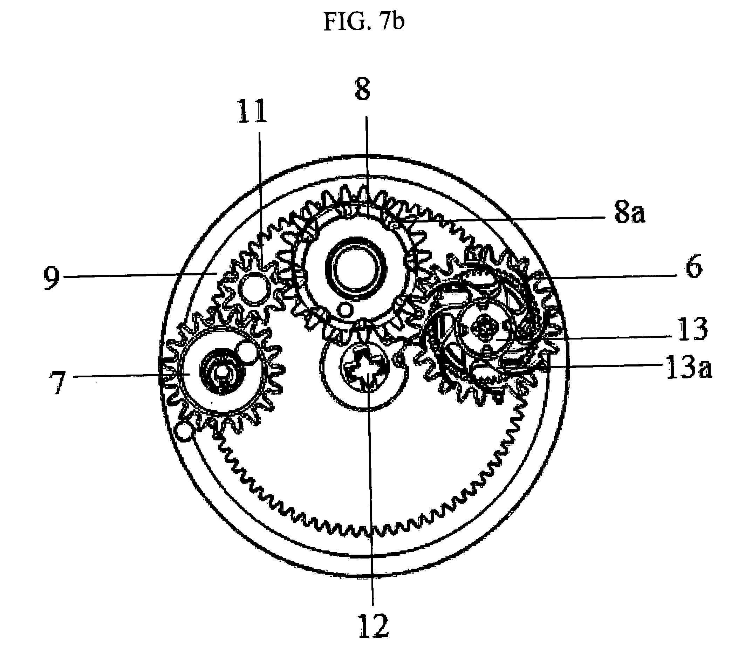

As can be seen in figure 7b, the indexing wheel (8) which synchronizes with the indexing ratchet wheel (3) is engaged with the winding wheel gear (6) and the pinion gear (11) and the rotation of the indexing wheel (8) causes the pinion gear (11) and the winding wheel gear (6) to rotate. Thus, both the peeled lid sheet (15b) of the blister package (15) which is indexed by the rotation of the indexing wheel (8) is tightly coiled on the winding wheel (13) engaging with the winding wheel gear (6) via the mechanism wheel (5) and also the counter wheel (9) is moved by the pinion gear (11) and the base gear (7) as a result of the rotation of the indexing wheel (8).

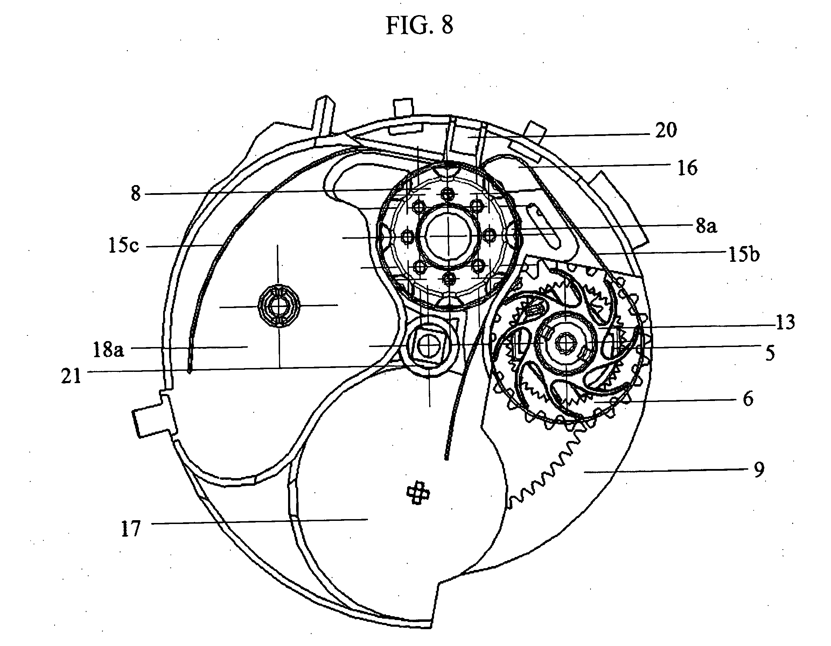

As is seen in figure 8, the lid sheet (15b) of the blister package (15) which is peeled away by the beak (16) and the base sheet (15c) are enclosed in separate compartments of the housing (10). The lid sheet (15b) that provides impermeability is indexed over the beak (16) and tightly coiled on the wings (13a) of the winding wheel. The base sheet (15c) of the blister package (15) where the blister pockets (15a) each of which carries one dose of the dry powder medicament are spaced is accumulated in the separated compartment (18a) of the housing (10). In response to each actuation of the device (1), one dose of the dry powder medicament which is prepared for inhalation after one blister pocket (15a) is opened and air entering the device through the air inlet (22) upon the inhalation of the patient provides to deliver one dose of the dry powder medicament to the patient by entraining it from the blister pocket (15a) to the mouthpiece (14).

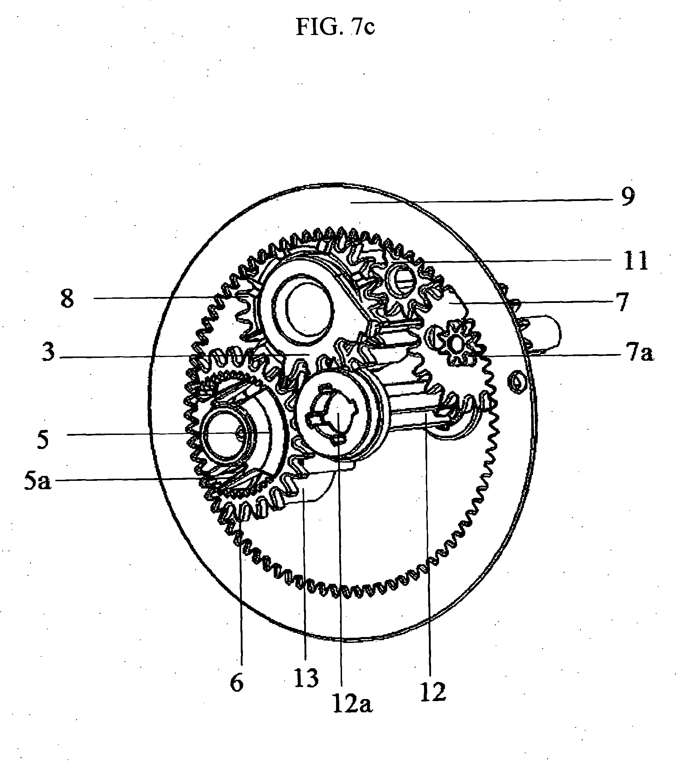

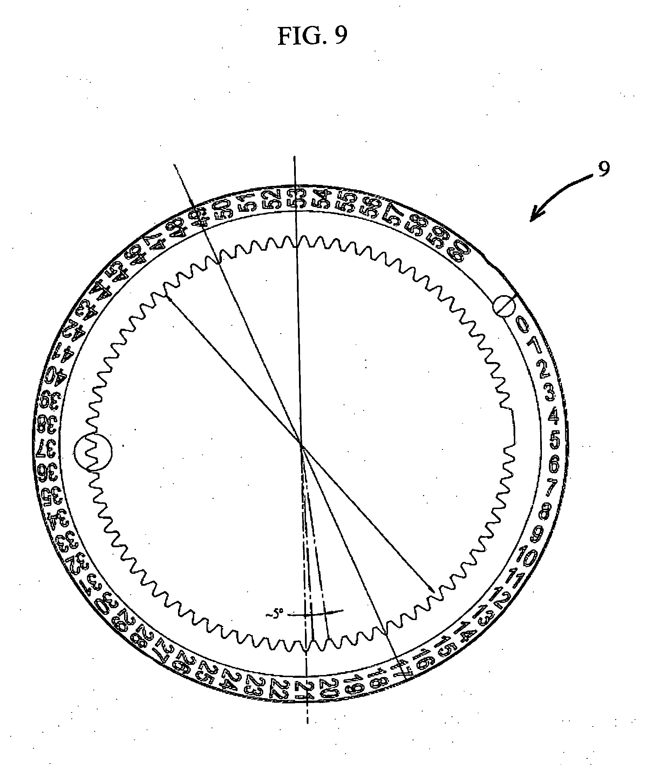

The rotation of the indexing wheel (8) is transmitted to the base gear (7) engaging with the pinion gear (11) by the pinion gear (11). The small gear which is under the base gear (7) as attached to it engages with the counter gear (9) (figure 7c). Thus, the movement of the indexing wheel (8) is transmitted to the counter wheel (9) shown in figure 9 by the pinion gear (11) and the base gear. There are numerals incrementing from 1 to 60 in the counter gear (9) displayed in figure 13. The angles between these numerals are all equal and approximately 5°. In response to each actuation of the device, the counter gear rotates approximately 5° and the number of the unused blister pockets remained in the device are clearly seen through the display aperture (4c) on the lower housing member (4b).

In use of the device described in figures 1-9, the mouthpiece (14) is exposed when the mouthpiece cover (2) is slid from the first position to the second position on the rotational path restricted on both ends by the protrusion parts (23a, 23b, 24a, 24b) on the upper housing member (4a) and the lower housing member (4b); the gear mechanism is triggered by the rotational movement of the mouthpiece cover (2) via the drive gear (12) and one dose of dry powder medicament is prepared for inhalation; the counter gear (9) is indexed and the numeral seen through the display aperture (4c) on the lower housing member (4b) is incremented. After inhalation, the mouthpiece cover (2) is moved from the second position to the first position wherein the mouthpiece (14) is completely covered.

The medicament in dry powder form which is stored in blister cavities is manufactured according to the prior art. According to the present invention, the particle sizes of the active agents comprised in the dry powder medicament are smaller than 20 µm, preferably smaller than 10 µm.

The inhaler pertaining to the present invention has been designed so as to deliver the dry powder medicament used in monotherapy or combined therapy. The term "monotherapy" refers to inhalation treatments in which dry powder medicaments comprising a single active agent are used whereas the term "combined therapy" refers to inhalation treatments in which dry powder medicaments comprising more than one active agents are use used.

The dry powder medicament delivered via the device of the present invention comprises at least one excipient in addition to the active agent or agents. These excipients are generally chosen from a group comprising monosaccharides (glucose, arabinose, etc.), disaccharides (lactose, saccharose, maltose, etc.), oligo- and polysaccharides (dextran, etc.), polyalcohols (sorbite, mannite, xylite), salts (sodium chloride, calcium carbonate, etc.) or combinations thereof. According to the present invention, the medicament in dry powder form comprises lactose as the excipient. The medicament in dry powder form comprises fine or coarse excipients particles preferably having various particle size ranges in order to deliver the required amount to the lungs.

The active agent or the active agents comprised in the dry powder medicament which is stored in blister packages used in the device pertaining to the present invention can be selected from a group comprising cromolyns, anti-infectives, antihistamines, anti-inflammatories, bronchodilators, leukotirene inhibitors, PED IV inhibitors, antitussives, diuretics, anticholinergics, hormones, xanthines and pharmaceutically acceptable combinations thereof.

The active agent comprised in the medicament in dry powder form delivered via the inhaler pertaining to the present invention is preferably selected from a group comprising tiotropium, oxitropium, flutropium, ipratropium, glicopironium, flunisolid, beclomethasone, budesonide, fluticasone, mometasone, ciclesonide, rofleponide, dexamethasone, montelukast, methylcyclopropane acetic acid, sodium cromoglicat, nedocromil sodium, Npropylene, teophylline, roflumilast, ariflo (cilomilast), salmeterol, salbutamol, formoterol, terbutaline, carmoterol, indacaterol, cetirizine, levocetirizine, efletirizine, fexofenadine and their racemates, free base, enantiomers or diastereomers and their pharmaceutically acceptable salts, solvates and/or hydrates or a combination of said active agents.

The device pertaining to the present invention is used in the administration of the medicament in dry powder form which is utilized in the treatment of many respiratory diseases, particularly in asthma, chronic obstructive pulmonary disorder (COPD) and allergic rhinitis. Accordingly, the respiratory diseases include, but not restricted to, allergic or non-allergic asthma at any phases, acute lung injury (ALI), acute respiratory distress syndrome (ARDS), exacerbation of airways hyperactivity, bronchiectasis, chronic obstructive pulmonary including emphysema and chronic bronchitis, airways or lung diseases (COPD, COAD or COLD), pneumoconiosis, aluminosis, anthracosis, asbestosis, chalicosis, ptilosis, siderosis, silicosis, tabacosis and byssinosis. The device pertaining to the invention can be used in prophylactic or symptomatic treatment. In addition, the medicament in dry powder form which is preferably used in the symptomatic treatment of allergic asthma and COPD is administered to the patient via the device pertaining to the present invention.

## Claims

1. An inhaler (1) suitable for delivery of a medicament in dry powder form from a blister package (15) composed of a plurality of blister pockets (15a) each of which comprises medicament in dry powder form and which are spaced at equal intervals, the inhaler comprising:
- a mouthpiece (14) enabling the patient to inhale a medicament in dry powder form from an opened blister pocket (15a),
- a gear mechanism enabling a blister package (15) to be indexed and the medicament in dry powder form to become ready for inhalation,
- a rotatable mouthpiece cover (2) covering the mouthpiece (14) and triggering the gear mechanism, and
- a housing (10) situated between an upper housing member (4a) and a lower housing member (4b) in which the blister package and the gear mechanism are enclosed
**characterized in that** said inhaler comprises stabilizing resilient covers (32, 33) that are connected with the upper and the lower housing members respectively (4a, 4b), and the movement of the mouthpiece cover (2) is hindered by pawls (32a, 33a) under the stabilizing resilient covers (32, 33) on connection points (29, 30) that enable the mouthpiece cover to communicate with the gear mechanism interlock with the mouthpiece cover (2) when the mouthpiece (14) is completely covered by the mouthpiece cover (2).

2. The inhaler (1) according to claim 1, wherein the rotation of the mouthpiece cover (2) is prevented on both sides by the pawls (32a; 33a) under each of the stabilizing resilient covers (32; 33) on both sides of said device interlock with the mouthpiece cover (2).

3. The inhaler (1) according to either one of claim 1 or claim 2, wherein the pawl (32a; 33a) under each stabilizing resilient cover (32; 33) raises and the mouthpiece cover (2) is released to trigger the gear mechanism when resilient parts of the stabilizing resilient covers (32d; 33d) are pressed.

4. The inhaler (1) according to any one of the preceding claims, wherein each stabilizing resilient cover (32; 33) is on the connection point (30; 29) of the mouthpiece cover.

5. The inhaler (1) according to any one of the preceding claims, wherein extensions (32b, 32c; 33b, 33c) under the stabilizing resilient covers (32,33) pass through the apertures (24c, 24d; 23c, 23d) on the upper or the lower housing member according to the position of the connection point (30; 29) and enable the stabilizing resilient covers (32; 33) that they connect with to remain stable.

6. The inhaler (1) according to claim 1, wherein the housing (10) comprises a beak (16) that enables the blister package (15) to be peeled.

7. The inhaler (1) according to claim 1, wherein a restricted path is provided by protrusions (23a, 23b; 24a, 24b) on the upper and lower housing members.

8. The inhaler (1) according to claim 1, wherein the mouthpiece cover (2) is rotationally moved by being slid on the upper (4a) and the lower (4b) housing member.

9. The inhaler (1) according to any one of the preceding claims, wherein the mouthpiece cover (2) can be in one of two positions:
- one where the mouthpiece (14) is completely covered and the device (1) is on standby mode when the mouthpiece cover (2) is leaning on a protrusion (23a, 24a) in one end of the rotational path,
- and the other where one dose of the medicament in dry powder form is ready for inhalation upon the actuation of the device (1) when the mouthpiece cover (2) leaning on a protrusion (23a, 24a) in the other end of the rotational path.

10. The inhaler (1) according to claim 1, wherein there is a carved part (2a) in one end of the mouthpiece cover (2) so as to allow the mouthpiece cover (2) to rotate easily.

11. The inhaler (1) according to claim 1, wherein all components of the gear mechanism directly or indirectly engage with each other.

12. The inhaler (1) according to either one of claim 1 or 11, wherein said gear mechanism is composed of;
- a drive gear (12) which actuate the device (1) by transmitting the constant-angle movement of the mouthpiece cover (2) to an indexing ratchet wheel (3);
- an indexing wheel (8) which synchronizes with the indexing ratchet wheel (3) and enables the blister package (15) to be indexed;
- a winding wheel gear (6) which moves a winding wheel (13) via a mechanism wheel (5) upon rotation of the indexing wheel (8);
- a pinion gear (11) and a base gear (7) that transmit the movement of the indexing wheel (8) to a counter wheel (9);
- wherein the counter wheel (9) displays the number of the unused blister pockets (15a) remaining in the device (1).

13. An inhaler according to any proceeding claim wherein the blister package (15) is composed of a plurality of blister pockets (15a), each of which comprises medicament in dry powder form and which are spaced at equal intervals.

## Patentansprüche

1. Ein Inhalator (1) geeignet für die Abgabe von Medikament in Form eines trockenen Pulvers aus einer Blisterverpackung (15) aus einer Vielzahl von Blister-Taschen (15a), von denen jede Medikament in Form eines trockenen Pulvers umfasst und die in gleichen Abständen angeordnet sind, wobei der Inhalator die Folgenden umfasst:
- ein Mundstück (14), das dem Patienten Medikament in trockener Pulverform aus einer geöffneten Blister-Tasche (15a) zu inhalieren ermöglicht,
- ein Getriebe, das einer Blisterverpackung (15) indiziert zu werden und das auch dem Medikamenten in trockener Pulverform zur Inhalation bereit zu werden ermöglicht,
- eine drehbare Mundstückabdeckung (2), die das Mundstück (14) verdeckt und das Getriebe auslöst, und
- ein Gehäuse (10), das sich zwischen einem oberen Gehäuseteil (4a) und einem unteren Gehäuseteil (4b) befindet, worin die Blisterverpackung und das Getriebe eingeschlossen sind
**dadurch gekennzeichnet, dass** das genannte Inhalator stabilisierende elastische Abdeckungen (32, 33) beinhaltet, die jeweils mit den oberen und unteren Gehäuseelementen (4a, 4b) verbunden sind, und die Bewegung der Mundstückabdeckung (2) durch Klinken (32a, 33a) unter den stabilisierenden elastischen Abdeckungen (32, 33) an Verbindungspunkten (29, 30) verhindert wird, die ermöglichen, dass der Mundstückabdeckung (2) mit der Getriebe-Verriegelung zu kommunizieren, wenn das Mundstück (14) vollständig durch das Mundstückabdeckung (2) abgedeckt wird.

2. Das Inhalator (1) nach Anspruch 1, wobei die Drehung der Mundstückabdeckung (2) auf beiden Seiten durch die Klinken (32a; 33a) verhindert wird, die sich unter jedem der stabilisierenden elastischen Abdeckungen (32; 33) auf beiden Seiten der Geräteverriegelung mit der Mundstückabdeckung (2) befinden.

3. Das Inhalator (1) nach einem der Ansprüche 1 oder 2, wobei die Klinke (32a; 33a) unter jeder stabilisierenden elastischen Abdeckung (32; 33) hebt und die Mundstückabdeckung (2) freigegeben wird, um das Getriebe auszulösen, wenn die elastischen Teile der stabilisierenden elastischen Abdeckungen (32d; 33d) gedrückt werden.

4. Das Inhalator (1) nach einem der vorhergehenden Ansprüche, wobei sich jede stabilisierende elastische Abdeckung (32; 33) an dem Verbindungspunkt (30; 29) der Mundstückabdeckung befindet.

5. Das Inhalator (1) nach einem der vorhergehenden Ansprüche, wobei Erweiterungen (32b, 32c; 33b, 33c) unter dem stabilisierenden elastischen Abdeckungen (32,33) durch die Öffnungen (24c, 24d; 23c, 23d) fallen, die sich in dem oberen oder dem unteren Gehäuseteil je nach Position der Verbindungsstelle (30; 29) befinden, und den stabilisierenden elastischen Abdeckungen (32; 33) ermöglichen, in Verbindung zu treten, um stabil zu bleiben.

6. Das Inhalator (1) nach Anspruch 1, wobei das Gehäuse (10) einen Schnabel (16) umfasst, der Blisterverpackung (15) ermöglicht, abgezogen zu werden.

7. Das Inhalator (1) nach Anspruch 1, wobei ein eingeschränkter Pfad durch Vorsprünge (23a, 23b; 24a, 24b) auf den oberen und unteren Gehäuseteilen vorgesehen ist.

8. Das Inhalator (1) nach Anspruch 1, wobei die Mundstückabdeckung (2) abwechselnd bewegt wird, indem sie auf dem oberen (4a) und dem unteren (4b) Gehäuseteil geschoben wird.

9. Das Inhalator (1) nach einem der vorhergehenden Ansprüche, wobei die Mundstückabdeckung (2) in einer von zwei Positionen sein kann:
- eine, wo das Mundstück (14) vollständig abgedeckt wird und die Vorrichtung (1) im Standby-Betrieb ist, wenn sich die Mundstückabdeckung (2) auf einen Vorsprung (23a, 24a) am einen Ende der Rotationsbahn lehnt,
- und die andere, wo eine Dosis des Medikamentes in trockener Pulverform bei der Betätigung der Vorrichtung (1) zur Inhalation bereit ist, wenn sich die Mundstückabdeckung (2) auf einen Vorsprung (23a, 24a) am anderen Ende der Rotationsbahn lehnt.

10. Das Inhalator (1) nach Anspruch 1, wobei es einen geschnitzten Teil (2a) am einen Ende der Mundstückabdeckung (2) gibt, so dass die Mundstückabdeckung (2) sich leicht drehen kann.

11. Das Inhalator (1) nach Anspruch 1, wobei alle Komponenten des Getriebes direkt oder indirekt miteinander in Eingriff stehen.

12. Das Inhalator (1) nach einem der Ansprüche 1 oder 11, wobei das genannte Getriebe die Folgenden umfasst;
- ein Antriebsgetriebe (12), das die Vorrichtung (1) durch Übertragen der konstanten Winkelbewegung der Mundstückabdeckung (2) zu einem Indizierungssperrrad (3) betätigt;
- ein Indizierungsrad (8), das mit dem Indizierungssperrrad (3) synchronisiert wird und der Blisterverpackung (15) indiziert zu werden ermöglicht;
- ein Wickelzahnrad (6), das ein Wickelrad (13) über ein Rad von Mechanismus (5) bei Drehung des Indizierungsrades (8) bewegt;
- ein Ritzel (11) und ein Basiszahnrad (7), die die Bewegung des Indizierungsrades (8) zu einem Gegenrad übermitteln (9);
- wobei das Gegenrad (9) die Anzahl der ungenutzten und in der Vorrichtung (1) verbleibenden Blister-Taschen (15a) zeigt.

13. Ein Inhalator nach einem der vorstehenden Ansprüche, wobei die Blisterverpackung (15) von einer Vielzahl von Blister-Taschen (15a) gebildet wird, von denen jedes Medikament in Form eines trockenen Pulvers umfasst und die in gleichen Abständen angeordnet sind.

## Revendications

1. Un inhalateur (1) approprié pour la livraison du médicament sous la forme de poudre sèche à partir d'un emballage coque (15) composée d'une pluralité de poches blisters (15a) dont chacune comprend le médicament sous la forme de poudre sèche et qui sont espacées à des intervalles égaux ; ledit inhalateur comprenant :
- un embout (14) permettant au patient d'inhaler un médicament sous la forme de poudre sèche depuis une poche blister ouverte (15a),
- un mécanisme d'engrenage permettant un emballage blister (15) à être indexé et le médicament sous la forme de poudre sèche à être prêt pour l'inhalation,
- une couverture rotative de l'embout (2) couvrant l'embout (14) et déclenchant le mécanisme d'engrenage, et
- un boîtier (10) situé entre l'élément de boîtier supérieur (4a) et l'élément de boîtier inférieur (4b), dans lequel l'emballage coque et le mécanisme d'engrenage sont enfermés
**caractérisé en ce que** ledit inhalateur comprend les couvertures résilientes stabilisantes (32, 33) qui sont connectées à l'élément de boîtier supérieur et inférieur respectivement (4a, 4b), et le mouvement de la couverture de l'embout (2) est empêché par les cliquets (32a, 33a) au-dessous des couvertures résilientes stabilisantes (32, 33) sur les points de connexion (29, 30) permettant la couverture de l'embout à communiquer la verrouillage du mécanisme d'engrenage avec la couverture de l'embout (2) quand l'embout (14) est couvert complètement par la couverture de l'embout (2).

2. L'inhalateur (1) selon la revendication 1, dans lequel la rotation de la couverture de l'embout (2) est empêchée de part et d'autre par les cliquets (32a; 33a) au-dessous de chacune des couvertures résilientes stabilisantes (32 ; 33) de part et d'autre du verrouillage d'appareil avec la couverture de l'embout (2).

3. L'inhalateur (1) selon quelconque des revendications 1 ou 2, dans lequel les cliquets (32a ; 33a) au-dessous de chacune des couvertures résilientes stabilisantes (32 ; 33) s'élèvent et la couverture de l'embout (2) est libéré pour déclencher le mécanisme d'engrenage quand les parties des couvertures résilientes stabilisantes (32d ; 33d) sont pressées.

4. L'inhalateur (1) selon quelconque des revendications précédentes dans lequel chacune des couvertures résilientes stabilisantes (32 ; 33) est sur le point de connexion (30, 29) de ladite couverture de l'embout.

5. L'inhalateur (1) selon quelconque des revendications précédentes, dans lequel les extensions (32b, 32c; 33b, 33c) au-dessous des couvertures résilientes stabilisantes (32 ; 33) se passent à travers des ouvertures (24c, 24d; 23c, 23d) sur l'élément de boîtier supérieur et inférieur selon la position dudit point de connexion (30 ; 29) et permettent les couvertures résilientes stabilisantes (32 ; 33) auxquelles elles sont connectées à rester stables.

6. L'inhalateur (1) selon la revendication 1, dans lequel le boîtier (10) comprend un onglet (16) qui permet l'emballage coque (15) à être pelé.

7. L'inhalateur (1) selon la revendication 1, dans lequel une route restreinte est prévue par les saillies (23a, 23b; 24a, 24b) sur les éléments de boîtier supérieur et inférieur.

8. L'inhalateur (1) selon la revendication 1, dans lequel la couverture de l'embout (2) est déplacée par rotation par glissement sur l'élément de boîtier supérieur (4a) et inférieur (4b).

9. L'inhalateur (1) selon quelconque des revendications précédentes dans lequel la couverture de l'embout (2) peut être dans une position sur deux :
- celle où l'embout (14) est couvert complètement et l'appareil (1) est en mode veille quand la couverture de l'embout (2) se penche sur une saillie (23a, 24a) en une extrémité de la route de rotation,
- et celle où une dose de médicament sous la forme de poudre sèche est prête pour l'inhalation sur la mise en marche de l'appareil (1) quand la couverture de l'embout (2) se penche sur une saillie (23a, 24a) en l'autre extrémité de la route de rotation.

10. L'inhalateur (1) selon la revendication 1, dans lequel il y a une partie gravée (2a) à une extrémité de la couverture de l'embout (2) afin de permettre la couverture de l'embout (2) à tourner facilement.

11. L'inhalateur (1) selon la revendication 1, dans lequel tous les composants du mécanisme d'engrenage s'engagent les uns avec les autres directement ou indirectement.

12. L'inhalateur (1) selon l'une quelconque des revendications 1 ou 11, dans lequel ledit mécanisme d'engrenage est composé de :
- un engrenage d'entrainement (12) qui enclenche l'appareil (1) en transmettant le mouvement d'un angle constant de la couverture de l'embout (2) à une roue à rochet d'indexation (3) ;
- une roue d'indexation (8) qui synchronise avec la roue à rochet d'indexation (3) et permet l'emballage coque (15) à être indexé ;
- un engrenage de roue d'enroulement (6) qui déplace une roue d'enroulement (13) par un mécanisme de roue (5) sur la rotation de la roue d'indexation (8) ;
- un engrenage à pignons (11) et un engrenage de base (7) qui transmettent le mouvement du la roue d'indexation (8) à une roue de contrepoussée (9) ;
- dans lequel la roue de contrepoussée (9) affiche le nombre des poches blisters (15a) inutilisées restantes dans l'appareil (1).

13. Un inhalateur selon quelconque des revendications précédentes dans lequel l'emballage coque (15) est composé d'une pluralité des poches blisters (15a), chacune desquelles comprenant le médicament sous la forme de poudre sèche et qui sont espacées à des intervalles égaux.
